# EUROPÄISCHE PATENTANMELDUNG

(11) **EP 2 444 231 A2**
(43) Veröffentlichungstag der Anmeldung: **25.04.2012**
(21) Anmeldenummer: 11186071.4
(22) Anmeldetag: 21.10.2011
(51) Int. Cl.: B29C 49/42, A61L 2/04

(54) **Vorrichtung und Verfahren zum Behandeln von Kunststoffbehältnissen mit konstantem Teilungsabstand**

(30) Priorität: 21.10.2010 DE 102010049029
(71) Anmelder: Krones AG, 93073 Neutraubling (DE)
(72) Erfinder: Voth, Klaus, 93083 Obertraubling (DE); Martini, Oliver, 3510 Konolfingen (CH); Lappe, Ulrich, 93059 Regensburg (DE); Söllner, Jürgen, 93176 Beratzhausen (DE); Winzinger, Frank, 93049 Regensburg (DE); Hausladen, Josef, 93086 Wörth/Donau (DE); Senn, Konrad, 93059 Regensburg (DE)
(74) Vertreter: Hannke, Christian

(57) **Zusammenfassung**

Die Erfindung betrifft eine Behälterbehandlungsmaschine (1) mit einer Streckblaseinrichtung (2) zum Umformen von Kunststoffbehältnissen (5), welche mindestens drei Transporteinrichtungen (4) zum Transportieren von Kunststoffbehältnissen (5) aufweist, wobei jede Transporteinrichtung (4) eine Vielzahl von Transportelementen (9) aufweist, welche jeweils an einem Träger (10) angeordnet sind und welche dazu geeignet sind, die Kunststoffbehältnisse (5) zu transportieren, wobei die Behälterbehandlungsmaschine einen Bereich (3) aufweist, welcher sich über mindestens drei Transporteinrichtungen (4) erstreckt, in welchem die Transportelemente (9) jeder der in diesem Bereich (3) angeordneten Transporteinrichtungen (4) in äquidistanten Abständen zu jeweils benachbarten Transportelementen (9) der selben Transporteinrichtung (4) angeordnet sind. Weiterhin betrifft die Erfindung ein Verfahren zum Transportieren von Kunststoffbehältnissen (5) in einer Behälterbehandlungsmaschine, insbesondere Streckblasmaschine, mittels einer Transporteinrichtung (4) mit einer Vielzahl von Transportelementen (9), welche an einem Träger (10) angeordnet sind und welche die Kunststoffbehältnisse (5) transportieren, wobei die Kunststoffbehältnisse (5) in einen sich über mindestens drei Transporteinrichtungen (4) erstreckenden Bereich (3) der Behälterbehandlungsmaschine mittels einer Vielzahl von in äquidistanten Abständen zu jeweils benachbarten Transportelementen (9) der selben Transporteinrichtung (4) angeordneten Transportelementen (9) so transportiert werden, dass der Abstand zwischen zwei benachbarten Kunststoffbehältnissen (5) konstant bleibt.

## Beschreibung

Die vorliegende Erfindung bezieht sich auf eine Behälterbehandlungsmaschine mit einer Streckblaseinrichtung zum Umformen von Kunststoffbehältnissen, welche mindestens drei Transporteinrichtungen zum Transportieren von Kunststoffbehältnissen aufweist, wobei jede Transporteinrichtung eine Vielzahl von Transportelementen aufweist, welche jeweils an einem Träger angeordnet sind und welche dazu geeignet sind, die Kunststoffbehältnisse zu transportieren, wobei die Behälterbehandlungsmaschine einen Bereich aufweist, welcher sich über mindestens drei Transporteinrichtungen erstreckt, in welchem die Transportelemente jeder der in diesem Bereich angeordneten Transporteinrichtungen in äquidistanten Abständen zu jeweils benachbarten Transportelementen der selben Transporteinrichtung angeordnet sind.

Weiterhin betrifft die Erfindung ein Verfahren zum Transportieren von Kunststoffbehältnissen in einer Behälterbehandlungsmaschine, insbesondere Streckblasmaschine, mittels einer Transporteinrichtung mit einer Vielzahl von Transportelementen, welche an einem Träger angeordnet sind und welche die Kunststoffbehältnisse transportieren, wobei die Kunststoffbehältnisse in einen sich über mindestens drei Transporteinrichtungen erstreckenden Bereich der Behälterbehandlungsmaschine mittels einer Vielzahl von in äquidistanten Abständen zu jeweils benachbarten Transportelementen der selben Transporteinrichtung angeordneten Transportelementen so transportiert werden, dass der Abstand zwischen zwei benachbarten Kunststoffbehältnissen konstant bleibt.

Der Teilungsabstand, also der Abstand zwischen zwei benachbarten Kunststoffbehältnissen, wird nach dem Stand der Technik im Verlauf des Behandlungsprozesses üblicherweise mehrfach verändert, um den Abstand benachbarter Kunststoffbehältnisse auf die Anforderungen folgender Behandlungseinrichtungen einzustellen. Bei dem den Teilungsabstand definierenden Abstand zwischen zwei benachbarten Kunststoffbehältnissen kann es sich um die kürzeste Strecke zwischen diesen Kunststoffbehältnissen handeln. Ebenso kann aber auch ein Abschnitt eines Umfanges der jeweiligen Behälterbehandlungseinrichtung oder ein Abschnitt des Transportpfades zur Ermittlung des Teilungsabstands herangezogen werden. In einer Vielzahl von Behälterbehandlungsmaschinen mit einer Streckblaseinrichtung zum Umformen von Kunststoffbehältnissen wird der Teilungsabstand beispielsweise vor dem Umformungsprozess auf den Teilungsabstand der Streckblaseinrichtung eingestellt. Dies geschieht üblicherweise mit sogenannten Teilungsverzugssystemen.

Die Begriffe "Behältnis", "Behälter" und "Kunststoffbehältnis" werden im Folgenden zur Vereinfachung synonym verwendet. Dabei schließen diese Begriffe auch Vorprodukte derartiger Behältnisse ein. Insbesondere beziehen sich diese Begriffe auch auf Vorformlinge (z.B. Kunststoffvorformlinge für z.B. Flaschen).

Einrichtungen zum Einstellen eines bestimmten Teilungsabstandes (im Folgenden auch als Abstandserzeugungseinrichtungen bezeichnet) können z.B. Transportschnecken sein. Unabhängig von der genauen Ausführungsvariante der Einrichtung zum Einstellen des gewünschten Teilungsabstandes können diese negative Einflüsse auf den gesamten Produktionsprozess haben oder einer möglichst raumsparenden Ausführung der gesamten Anlage entgegen stehen.

Jede Einrichtung zum Einstellen des gewünschten Teilungsabstandes erfordert ein - wenn auch im besten Fall sehr kleines - Reservoir an Kunststoffbehältnissen, die stromabwärts der Abstandserzeugungseinrichtung in einem vorgegebenen Abstand abgegeben werden sollen. Idealerweise ist die Anzahl an Kunststoffbehältnissen, welche der Abstandserzeugungseinrichtung zugeführt werden, gleich der, welche von der Abstandserzeugungseinrichtung befördert werden können und auch identisch mit der Anzahl an Kunststoffbehältnissen, welche von der stromabwärts angeordneten Behandlungseinrichtung behandelt werden können. Aufgrund der unterschiedlichen Teilungsabstände einzelner Behandlungseinrichtungen kommt es jedoch zu geringfügigen Unterschieden in der jeweiligen Kapazität der einzelnen Behandlungseinrichtungen, wodurch im Laufe des Produktionsprozesses Unregelmäßigkeiten auftreten, welche durch zwischengeschaltete Puffer ausgeglichen werden müssen.

Diese Puffer oder Zwischenlager beanspruchen zusätzlichen Platz und stehen so einer möglichst kompakten und platzsparenden Ausführung einer Behälterbehandlungsmaschine mit einer Streckblaseinrichtung zum Umformen von Kunststoffbehältnissen entgegen.

Weiterhin ist es vergleichsweise aufwändig, derartige Puffer, insbesondere jedoch die Abstandserzeugungseinrichtungen steril zu halten. Durch die gestiegenen Anforderungen im Bereich der Sterilität von Kunststoffbehältnissen für die Verpackung, insbesondere von Medikamenten und Lebensmitteln, sind für das Sterilhalten von Abstandserzeugungseinrichtungen besonders aufwändige und kostenintensive Verfahren und Vorrichtungen notwendig. Beispielsweise kann die gesamte Behälterbehandlungsmaschine in einem sehr großen Reinraum angeordnet werden. Dies ist jedoch in vielen Fällen unwirtschaftlich, da einerseits ein unnötig großer Bereich steril gehalten werden muss und andererseits bei Wartungsarbeiten zwangsläufig der gesamte sterile Bereich geöffnet werden muss und somit kontaminiert wird.

Aufgabe der Erfindung ist es daher, eine Behälterbehandlungsmaschine mit einer Streckblaseinrichtung zum Umformen von Kunststoffbehältnissen bereitzustellen, in welcher eine Vielzahl von Transporteinrichtungen zum Transportieren von Kunststoffbehältnissen Transportelemente aufweist, welche jeweils in äquidistanten Abständen zu jeweils benachbarten Transportelementen der selben Transporteinrichtung angeordnet sind.

Weiterhin ist es Aufgabe der Erfindung, ein Verfahren zum Transportieren von Kunststoffbehältnissen in einer Behälterbehandlungsmaschine mittels einer Transporteinrichtung mit einer Vielzahl von Transportelementen, bereitzustellen wobei die Kunststoffbehältnisse in einem sich über eine Vielzahl von Transporteinrichtungen erstreckenden Bereich der Behälterbehandlungsmaschine so transportiert werden, dass der Abstand zwischen zwei benachbarten Kunststoffbehältnissen konstant bleibt.

Diese Aufgaben werden erfindungsgemäß durch die Gegenstände der unabhängigen Patentansprüche gelöst.

Ein wesentlicher Aspekt der Erfindung ist eine Behälterbehandlungsmaschine mit einer Streckblaseinrichtung zum Umformen von Kunststoffbehältnissen, welche mindestens drei Transporteinrichtungen zum Transportieren von Kunststoffbehältnissen aufweist, wobei jede Transporteinrichtung eine Vielzahl von Transportelementen aufweist, welche jeweils an einem Träger angeordnet sind und welche dazu geeignet sind, die Kunststoffbehältnisse zu transportieren, wobei die Behälterbehandlungsmaschine einen Bereich aufweist, welcher sich über mindestens drei Transporteinrichtungen erstreckt, in welchem die Transportelemente jeder der in diesem Bereich angeordneten Transporteinrichtungen in äquidistanten Abständen zu jeweils benachbarten Transportelementen der selben Transporteinrichtung angeordnet sind.

Vorzugsweise schließen sich die drei Transporteinrichtungen innerhalb dieses Bereichs unmittelbar aneinander an, d.h., die Behältnisse werden von einer dieser Transporteinrichtungen unmittelbar an die nächste übergeben.

Es wird dabei darauf hingewiesen, dass es sich bei den Transporteinrichtungen auch um diejenigen Transporteinrichtungen handeln kann, welche die Behältnisse während des Blasvorgangs transportieren, allgemein gesagt, welche die Behältnisse während einer Behandlung, zum Beispiel eines Blasens, Füllens oder Sterilisierens, transportieren.
Weiterhin kann unter einer Behälterbehandlungsmaschine neben einer Streckblasmaschine, auch ein Füller, ein Verschließer, eine Ettiketier- bzw. Sterilisiermaschine verstanden werden bzw. auch mehrere der genannten Maschinen zusammen.

Durch eine derartige Behälterbehandlungsmaschine ist es möglich, dass in dem Bereich, in welchem die Transportelemente jeder der in diesem Bereich angeordneten Transporteinrichtungen in äquidistanten Abständen zu jeweils benachbarten Transportelementen der selben Transporteinrichtung angeordnet sind, alle Kunststoffbehältnisse mit der exakt gleichen (insbesondere transversalen) Geschwindigkeit bewegt werden und so die Übergabe der Kunststoffbehältnisse zwischen den benachbarten Transporteinrichtungen vereinfacht wird.

Bei der Geschwindigkeit der Kunststoffbehältnisse kann es sich beispielsweise um die Bahngeschwindigkeit, Umfangsgeschwindigkeit, Tangentialgeschwindigkeit oder ähnliche handeln. Im Wesentlichen kreisförmige, um eine zentrale Achse rotierende Transporteinrichtungen mit einer geringeren Anzahl an Transportelementen (und damit aufgrund des gleichen Abstandes zwischen den Transportelementen zwangsläufig geringerem Radius) rotieren daher, um die selbe Tangentialgeschwindigkeit an den Positionen der Kunststoffbehältnisse zu erreichen, mit einer größeren Winkelgeschwindigkeit als Transporteinrichtungen mit größerem Radius und somit einer größeren Anzahl an Transportelementen.

Durch den über mehrere Transporteinrichtungen gleichbleibenden Teilungsabstand wird gewährleistet, dass während der Zeit, die für die Übergabe des Kunststoffbehältnisses benötigt wird, dieses in dem Augenblick, in dem es mit beiden Transportelementen in Kontakt steht, aufgrund der identischen Geschwindigkeit der Transportelemente, keinen oder nur äußerst geringen Spannungen aufgrund unterschiedlicher Bewegungsgeschwindigkeiten der Transportelemente ausgesetzt sind. Ebenso können auch ungleichmäßige Bewegungen der an der Übergabe beteiligten Transporteinrichtungen reduziert werden, da die Transporteinrichtungen sich mit der selben Tangentialgeschwindigkeit bewegen, und somit keine durch die andere schnellere oder langsamere Transporteinrichtung beschleunigt oder abgebremst wird. Dementsprechend wird die Synchronisation innerhalb eines Verbundes einer Vielzahl von Transporteinrichtungen in einer Behälterbehandlungsmaschine deutlich vereinfacht. Asynchronitäten können reduziert und/oder schnell und einfach ausgeglichen werden.

Für viele Medikamente und Lebensmittel, wie z.B. Getränke, ist es erforderlich, sie unter aseptischen Bedingungen abzufüllen. Dazu ist es nach dem Stand der Technik möglich, dass sich die Behälterbehandlungsmaschine in einem Reinraum befindet und zugeführte Kunststoffbehältnisse vor dem Einschleusen in diesen Reinraum einem Sterilisationsprozess unterzogen werden. Da die Anordnung der gesamten Behälterbehandlungsmaschine in dem Reinraum ein äußerst großes steril zu haltendes Volumen benötigt und damit sehr kostenintensiv ist, wurden Behälterbehandlungsmaschinen entwickelt, welche den Reinraum auf einen Kanal reduzieren, welcher sich entlang des Transportpfades der Kunststoffbehältnisse erstreckt. Für eine Vielzahl von Behandlungseinrichtungen wie z.B. Transporteinrichtungen, Streckblaseinrichtungen usw. wurden Konstruktionen entwickelt, bei denen ein Großteil der jeweiligen Behandlungseinrichtung unsteril und damit einfach zugänglich gestaltet ist und sich nur die Behandlungselemente in dem sterilen Kanal oder Tunnel entlang des Transportpfades der Kunststoffbehältnisse befinden. Die Behandlung erfolgt somit im sterilen Bereich.

In einer besonders bevorzugten Ausführungsform der Behälterbehandlungsmaschine ist der Bereich ein Reinraum.

Das Sterilisieren der Behältnisse vor der Befüllung mit sensiblem Füllgut kann beispielsweise derart erfolgen, dass die leeren Behältnisse vor dem Füllvorgang separat entkeimt werden. In einem nachgeschalteten Prozessschritt werden diese unter aseptischen Bedingungen mit an anderer Stelle sterilisiertem Füllgut befüllt. Die Sterilität des leeren Behältnisses wird dabei durch chemische Desinfektionsmittel wie beispielsweise Peroxide oder Persäuren oder andere erreicht. Dazu werden die Behältnisse einem sogenannten Isolator zugeführt, in dem sie mit dem Desinfektionsmittel beaufschlagt werden. Das Desinfektionsmittel wirkt eine bestimmte Zeit ein und wird anschließend mit verhältnismäßig hohem Aufwand wieder entfernt.

Eine andere Möglichkeit besteht darin, nicht die Kunststoffflasche selbst zu sterilisieren, sondern bereits den Kunststoffvorformling. Ein Kunststoffvorformling weist eine erheblich geringere zu sterilisierende Oberfläche auf, wodurch es bei dessen Sterilisation ermöglicht wird, im Vergleich zur Sterilisation der fertigen Flasche, Sterilisationsmittel einzusparen. Allerdings ist es bei diesem Verfahren notwendig, die Behältnisse nach deren Sterilisation, über den gesamten weiteren Transportweg, über den Blas- und den Abfüllvorgang hinaus, unter sterilen Bedingungen zu transportieren. Mindestens bis zum Verschließen der befüllten Behälter, müssen die aseptischen Bedingungen aufrecht erhalten werden.

In einer bevorzugten Ausführungsform der Behälterbehandlungsmaschine weisen eine Transporteinrichtung und eine stromabwärts unmittelbar folgende Streckblaseinrichtung Transportelemente in äquidistanten Abständen zu jeweils benachbarten Transportelementen auf. Dadurch ist es möglich, die Kunststoffvorformlinge bereits mit dem an der Streckblaseinrichtung benötigten Teilungsverzug an diese zu Übergeben. Eine separate Abstandserzeugungseinrichtung zur Einstellung des Teilungsverzugs direkt vor dem Blasprozess ist damit nicht notwendig.

Zum Erzeugen einer Flasche aus einem Kunststoffvorformling ist es notwendig, dass der Kunststoffvorformling erwärmt wird. Daher umfasst die Behälterbehandlungsmaschine in einer bevorzugten Ausführungsform mindestens eine Heizeinrichtung zum Erwärmen der Kunststoffbehältnisse. Eine derartige Heizeinrichtung befindet sich bevorzugt stromaufwärts der Umformungseinrichtung, bevorzugt der Streckblaseinrichtung. Vorzugsweise weist eine Streckblasmaschine wenigstens eine Blasstation auf und vorteilhaft eine Vielzahl von Blasstationen. Vorteilhaft weisen die Blasstationen jeweils Reckstangen auf, um die Kunststoffvorformlinge entlang deren Längsrichtung zu dehnen.

In einer weiteren bevorzugten Ausführungsform der Behälterbehandlungsmaschine umfasst die Behälterbehandlungsmaschine mindestens eine Sterilisationseinrichtung zum Sterilisieren der Kunststoffbehältnisse.

Weiterhin kann die Behälterbehandlungsmaschine weitere Behandlungseinrichtungen wie Blaseinrichtungen, Abfülleinrichtungen, Verschließeinrichtungen, Etikettiervorrichtungen, Transportsterne usw. umfassen.

Bei den Behältnissen handelt es sich insbesondere um Kunststoffbehältnisse wie insbesondere Kunststoffvorformlinge oder aus diesen Kunststoffvorformlingen hergestellte Kunststoffflaschen. Die Erfindung kann jedoch auch für andere Behältnisse wie beispielsweise Glasflaschen, Dosen oder Kartonagen Anwendung finden.

Die Steuerung der Geschwindigkeiten, mit denen sich die Kunststoffbehältnisse jeweils bewegen, lassen sich besonders leicht bei einer im Wesentlichen kreisförmigen Behandlungseinrichtung steuern. Durch eine Änderung der Winkelgeschwindigkeit (und somit der Anzahl der Umdrehungen pro Zeiteinheit) kann direkt auch die Tangentialgeschwindigkeit (und somit die Geschwindigkeit der Kunststoffbehältnisse) gesteuert werden. In einer bevorzugten Ausführungsform der Behälterbehandlungsmaschine ist mindestens eine der Transporteinrichtungen dazu geeignet, die Kunststoffbehältnisse entlang eines Abschnittes eines weitgehend kreisrunden Transportpfades zu transportieren. Dazu kann z.B. der Umfang einer Transporteinrichtung im Wesentlichen kreisrund sein. Ebenso ist es möglich, dass die Transporteinrichtung eine von der Kreisform abweichende Geometrie aufweist, jedoch die Transportelemente derart angeordnet sind, dass die Aufnahmeelemente für die Kunststoffbehältnisse sich auf einer Kreisbahn bewegen. Bevorzugt ist wenigstens eine der Transporteinrichtungen eine Transporteinrichtung, welche der Streckblaseinrichtung die Kunststoffvorformlinge zuführt.

Bei dieser in etwa kreisförmigen Ausführung der einzelnen Behandlungseinrichtungen ist es für eine möglichst einfache Übergabe der einzelnen Kunststoffbehältnisse von einer Behandlungseinrichtung zur nächsten stromabwärts befindlichen Behandlungseinrichtung vorteilhaft, wenn diese Behandlungseinrichtungen in entgegengesetzte Richtungen rotieren. Dabei sind bei gleicher Anordnung der Behandlungseinrichtungen grundsätzlich zwei verschiedene Transportpfade möglich. In einer bevorzugten Ausführungsform der Behälterbehandlungsmaschine umfasst ein Transportpfad, entlang welchem die Kunststoffbehältnisse transportierbar sind, einen Abschnitt des Umfanges mindestens einer Transporteinrichtung, welcher durch einen Winkel von über 180° aufgespannt ist. Durch diese Ausführungsform ist es möglich, dass sich die Kunststoffbehältnisse lange im Einflussbereich einer bestimmten Behandlungseinrichtung befinden und so ein vergleichsweise langer Zeitraum für die Durchführung der jeweiligen Behandlung zur Verfügung steht. Da ein solcher längerer Zeitraum für eine Vielzahl von Prozessschritten notwendig sein kann, ist der Transportpfad bevorzugt zumindest abschnittsweise in etwa mäanderförmig ausgebildet.

In anderen Worten ist der Transportpfad eines Behältnisses beim Transport von mindestens zwei Transporteinrichtungen mäanderförmig.

Auf diesem Transportpfad werden die Kunststoffbehältnisse durch die Transport- und/oder Behandlungseinrichtungen geführt. Insbesondere weisen die Transport- und/oder Behandlungseinrichtungen dazu Transportelemente auf, die in der Lage sind, jeweils ein Kunststoffbehältnis aufzunehmen und auf dem Transportpfad vorwärts zu bewegen. Ebenso ist es möglich, dass eine Transport- und/oder Behandlungseinrichtung mehrere Kunststoffbehältnisse bewegt. Bevorzugt sind die Transportelemente derart ausgebildet, dass sie das Kunststoffbehältnis zumindest abschnittsweise z.B. mittels Greifwerkzeugen, welche zwei Greifklauen aufweisen und das Kunststoffbehältnis im sogenannten Neckhandling greifen, umfassen.

Die Transportelemente sind dabei bevorzugt als aktive Transportelemente ausgebildet, welche das jeweilige Kunststoffbehältnis an einer Übergabeposition gezielt aufnehmen und fixieren können und an einer stromabwärtsbefindlichen weiteren Übergabeposition erneut durch einen aktiven Prozess wieder gezielt an eine andere Transport- und/oder Behandlungseinrichtung abgeben können. In einer bevorzugten Ausführungsform der Behälterbehandlungsmaschine ist mindestens eines der Transportelemente in seinem Öffnungsquerschnitt zum Einführen und/oder Greifen eines Kunststoffbehältnisses veränderbar.

Bei einer weiteren vorteilhaften Ausführungsform stehen die Abstände der Transportelemente einer ersten in dem Bereich angeordneten Transporteinrichtung in einem ganzzahligen Verhältnis zu den Abständen der Transportelemente einer zweiten in diesem Bereich angeordneten Transporteinrichtung. Dabei ist es möglich, dass die Abstände jeweils genau gleich sind, d.h. der ganzzahlige Faktor den Wert 1 hat, es wäre aber auch möglich, dass die Abstände der Transportelemente bei einer Transporteinrichtung doppelt, dreimal, viermal, fünfmal... n-mal so groß wie die Abstände bei einer weiteren Transporteinrichtung sind.

Besonders bevorzugt weisen wenigstens zwei Transporteinrichtungen in dem Bereich während eines Arbeitsbetriebs unterschiedliche und in einem ganzzahligen (insbesondere konstanten) Verhältnis zueinander stehende Transportgeschwindigkeiten auf.

Vor der Abfüllmaschine kann es jedoch auch vorkommen, dass aus Bauraumgründen der Abstand verändert werden muss. Dies kann mittels einer gut reinig- und sterilisierbaren Einteilschnecke geschehen. Um den Abstand auch hier gleich zu lassen, wäre es denkbar, auf den unterschiedlichen Transporteinrichtungen und insbesondere auf denen, auf denen die Behältnisse behandelt werden, ein Verfahren einzusetzen, nach dem sich ein Behältnis zwei oder mehrmals auf der selben, rundlaufenden Transporteinrichtung mitdreht, bevor es aus dieser entnommen wird. So kann man die Behandlungszeit steigern, ohne den Abstand verändern zu müssen. Ein solches Verfahren ist aus der Druckschrift EP 1 687 222 B1 bekannt. Der Inhalt dieser Druckschrift wird durch Bezugnahme auch vollständig zum Inhalt der vorliegenden Anmeldung gemacht. Diese Vorgehensweise kann auch bei der Sterilisation von Kunststoffvorformlingen Anwendung finden.

Bei einer Aseptischen Blasmaschine tritt das Problem auf, dass ein Teilungsverzugsstern zwischen Sterilisationsmodul und Blasmaschine schwer technisch ausführbar ist.
Auch in diesem Fall ist es möglich, den Teilungsverzug vor dem Zwischenmodul zu vollziehen. Da die Teilung der Blasmaschine aber sehr groß ist z.B. 600mm müsste sie auf dem Zwischenmodul auch 600mm betragen dies würde aber ein sehr großes Zwischenmodul erfordern.

Um dies zu umgehen, kann man das Zwischenmodul nun so auslegen das es eine 120-Teilung hat und nur jede 5. Station pro Umdrehung bestückt wird. Wenn nun das Zwischenmodul 5*n+1 Stationen hat und es sich 5 mal schneller als das Blasrad dreht, ist es möglich, ohne Teilungsverzug die Übergabe der Behältnisse zwischen der Blaseinrichtung und dem Zwischenmodul zu gewährleisten.

Dabei ist hier auch eine punktuelle Übergabe mittels einer Klammer an der Blasform denkbar

Wenn nun die Blasform eine Art Klammer oder Fangtasche bekommt, und somit die Blasform bei den Übergaben nicht mehr begleitet werden muss, kann der Übergabestern als fester Stern ausgeführt werden, welcher einfach in ein Aseptikkonzept zu integrieren ist. Bei aus dem Stand der Technik bekannten Vorrichtungen übernimmt die Blasform selbst den Kunststoffvorformling. Dies bedeutet, dass die Transporteinrichtung, welche den Kunststoffvorformling an die Blasform übergibt, diesen Kunststoffvorformling über eine gewisse Wegstrecke hinweg mit der Blasform mitführen muss, um die Übergabe zu gewährleisten. Dies ist jedoch relativ aufwändig und insbesondere weicht hier der Transportpfad von einer Greifbahn ab. Bei der hier beschriebenen Vorgehensweise hingegen gibt die Transporteinrichtung den Kunststoffvorformling an die betreffende Greifklammer der Blaseinrichtung ab und erst nach dieser Übergabe schließt sich die Blasform um den Kunststoffvorformling.

Vorzugsweise wird die Blasform hier weiter geöffnet als bei aus dem Stand der Technik bekannten Vorrichtungen.

Ein weiterer wesentlicher Aspekt der Erfindung ist ein Verfahren zum Transportieren von Kunststoffbehältnissen in einer Behälterbehandlungsmaschine, insbesondere Streckblasmaschine, mittels einer Transporteinrichtung mit einer Vielzahl von Transportelementen, welche an einem Träger angeordnet sind und welche die Kunststoffbehältnisse transportieren, wobei die Kunststoffbehältnisse in einem sich über mindestens drei Transporteinrichtungen erstreckenden Bereich der Behälterbehandlungsmaschine mittels einer Vielzahl von in äquidistanten Abständen zu jeweils benachbarten Transportelementen der selben Transporteinrichtung angeordneten Transportelementen so transportiert werden, dass der Abstand zwischen zwei benachbarten Kunststoffbehältnissen konstant bleibt. Die Behältnisse werden innerhalb dieses Bereiches somit ohne Änderungen des Teilungsverzuges transportiert.

Durch dieses Verfahren können Behälter unter Reduzierung der mit der Übergabe der Behältnisse zwischen einzelnen Behandlungseinrichtungen verbundenen Probleme transportiert werden und gleichzeitig das erneute Einstellen eines bestimmten Teilungsabstandes im Verlauf des Transportes entlang eines Abschnittes des Transportpfades vermeiden werden. Außerdem ist es möglich, den Transport zu beschleunigen, da der Transportpfad aufgrund des Wegfalls von Abstandserzeugungseinrichtungen verkürzt werden kann. Dadurch fallen auch Zwischenreservoire weg, in welchen die Kunststoffbehältnisse vor der erneuten Einstellung eines Teilungsabstandes zwischengelagert werden.

In einer bevorzugten Variante des Verfahrens werden die Kunststoffbehältnisse in einem Bereich der Behälterbehandlungsmaschine, welcher eine Heizeinrichtung für Kunststoffbehältnisse aufweist, in einem konstanten, bevorzugt äquidistanten Abstand, entlang eines Transportpfades transportiert. Durch diese Variante, in der der Bereich, in dem der Teilungsabstand konstant bleibt die Heizeinrichtung einschließt, ist es möglich, dass die durch die Heizeinrichtung erwärmten Behältnisse direkt an die Streckblaseinrichtung weitergegeben werden. Demnach muss der Streckblaseinrichtung keine separate Abstandserzeugungseinrichtung vorgeschaltet werden, welche die bereits erwärmten Behältnisse evtl. beschädigen könnte.

Bei einer besonders bevorzugten Variante des Verfahrens werden die Kunststoffbehältnisse innerhalb des Bereiches unter sterilen Bedingungen innerhalb eines Reinraums, transportiert. Durch diese Variante kann vermieden werden, dass durch eine (oder eine zusätzliche) Abstandserzeugungseinrichtung sich innerhalb des Reinraums ein möglicher zusätzlicher Herd für Kontaminationen befindet und die Kunststoffbehältnisse durch diesen verunreinigt werden.

In einer weiteren bevorzugten Variante des Verfahrens wird ein Abstand zwischen den Kunststoffbehältnissen stromabwärts des Bereiches in dem der Abstand zwischen zwei benachbarten Kunststoffbehältnissen während des Transportes über mehrere Transporteinrichtungen konstant bleibt, mittels einer Abstandserzeugungseinrichtung, bevorzugt mittels einer Schnecke, verändert. Nach dem Verlassen des Bereiches in dem der Abstand zwischen zwei benachbarten Kunststoffbehältnissen während des Transportes konstant bleibt ist es möglich, dass für spezielle Behandlungsschritte veränderte Abstände benötigt werden. Ein Beispiel für einen solchen Behandlungsschritt ist das Verpacken mehrerer befüllter, etikettierter und verschlossener Behältnisse in einer gemeinsamen größeren Verpackungseinheit. Dabei sollten die Behältnisse möglichst platzsparend angeordnet sein, so dass der Abstand für den Verpackungsprozess möglichst reduziert werden sollte. Andere mögliche Prozessschritte bei denen ein neu eingestellter und evtl. abweichender Abstand zwischen einzelnen Behältnissen vorteilhaft sein kann, kann beispielsweise das Etikettieren, eine Füllstandskontrolle, Qualitätssicherung oder ein anderer Prozessschritt sein. Dabei kann prinzipiell jede nach dem Stand der Technik bekannte Möglichkeit der Einstellung eines vorteilhaften Abstandes genutzt werden. Als besonders vorteilhaft haben sich jedoch Schnecken erwiesen.

In einer bevorzugten Ausführungsform wird der äquidistante Abstand zwischen den Behältnissen auf den Transporteinrichtungen in der Reihenfolge des durchlaufenen Transportpfads des Behältnisses
- Vorformlingssterilisation
- Übergabestern zu der Streckblasvorrichtung
- Streckblasvorrichtung
- Entnahmestern zum Entnehmen der Behälter aus der Streckblasvorrichtung durchweg eingehalten. Zusätzlich können vor der Vorformlingssterilisation in einem Einstufenverfahren eine rundlaufende Spritzgussmaschine zur Produktion von Vorformlingen, eine Temperiervorrichtung für die frisch gespritzten Vorformlinge oder eine Heizvorrichtung für Vorformlinge, die im Zweistufenverfahren eingesetzt werden, vorgesehen sein, auf denen der äquidistante Abstand eingehalten wird. Zusätzlich können nach dem Entnahmestern eine Reinigungs- oder Sterilisationsvorrichtung, eine Etikettiermaschine, eine Abfüllmaschine und/oder ein Verschließer vorgesehen sein, auf denen der äquidistante Abstand ebenfalls eingehalten wird. Insbesondere kann vom Spritzmodul, jedoch spätestens von der Vorformlingssterilisation an ein Reinraum vorgesehen sein, welcher sich auch dadurch auszeichnet, dass in diesem ein Überdruck im Vergleich zu anschließenden Prozesskammern und/oder zur Umgebung herrscht, indem sterile Luft eingeblasen wird.

Insbesondere ist der Reinraum im Bereich der Blasmaschine sehr klein gehalten. Dies kann durch einen schlauchförmig ausgeprägten Reinraum realisiert werden. Bei rundlaufenden Transporteinrichtungen ist dieser in anderen Worten im Wesentlichen torusförmig ausgeprägt. In einem Reinraum sind hierbei nur die für die Behandlung und den Transport nötigsten mechanischen Vorrichtungen vorgesehen, um die Reinigung/Sterilisation des Reinraums in einem Cleaning - in - place - Verfahren zu erleichtern. Um trotzdem eine Bewegbarkeit der Behandlungs- und Transportelemente zu gewährleisten sind oftmals Faltenbalge als Abdichtung der Behandlungs- und Transportelemente zu den Reinraumwänden vorgesehen. Bei der Nutzung des erfindungsgemäßen Verfahrens können die Faltenbalge sehr einfach gehalten werden, da durch den nicht vorhandenen Teilungsverzug keine komplizierten Bewegungsabläufe der Transportelemente stattfinden müssen. Ein Beispiel für einen komplizierten Bewegungsablauf ist eine kombinierte Linear- und Drehbewegung eines Greifelements, was in den Dokumenten DE 3837118 A1 oder DE 3109267 A1 beschrieben wird. Bei den Transporteinrichtungen, insbesondere im Bereich der Abfüllmaschine, kann auch ein Reinraum vorgesehen sein, der mit einem hausähnlichem Maschinenschutz abschließt. Der Reinigungsaufwand ist hier etwas höher.

Weitere Vorteile und Ausführungsformen ergeben sich aus den beigefügten Zeichnungen:

Darin zeigen:
- Fig. 1: eine schematische Darstellung einer Behälterbehandlungsmaschine mit einer Streckblaseinrichtung zum Umformen von Kunststoffbehältnissen mit einem Reinraum, innerhalb dessen der Teilungsabstand der behandelten Kunststoffbehältnisse über mehrere Behandlungseinrichtungen konstant bleibt;
- Fig. 2: eine weitere schematische Darstellung einer Behälterbehandlungsmaschine ähnlich wie in Fig. 1, bei der jedoch der Teilungsabstand bereits vor der Einbringung der Kunststoffbehältnisse in den Reinraum eingestellt wird;
- Fig. 3: eine schematische Darstellung einer Behälterbehandlungsmaschine, bei der der mäanderförmige Transportpfad besonders hervorgehoben ist.

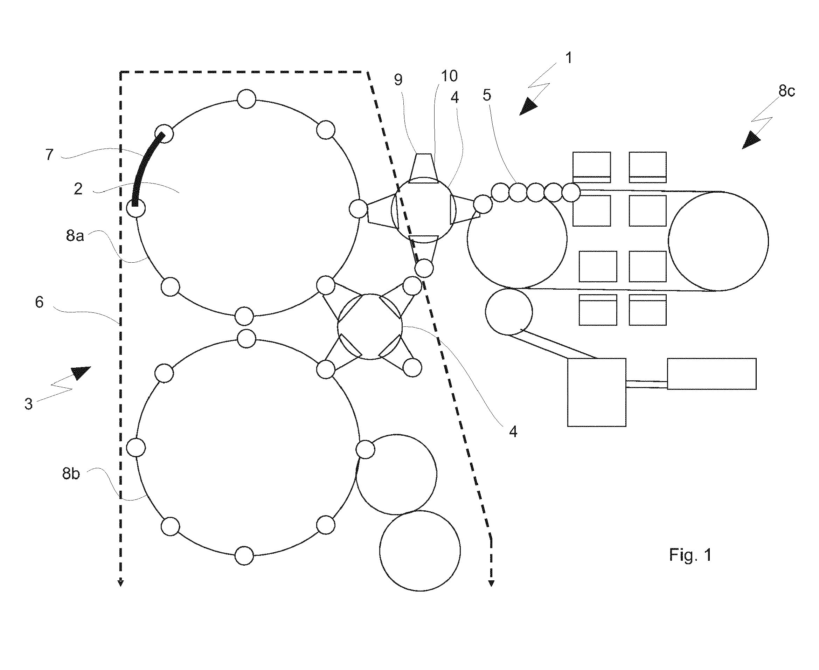

Figur 1 zeigt eine schematische Darstellung einer Behälterbehandlungsmaschine 1 mit einer Streckblaseinrichtung 2 zum Umformen von Kunststoffbehältnissen 5 mit einem Reinraum 6, innerhalb dessen der Teilungsabstand 7 der behandelten Kunststoffbehältnisse 5 über mehrere Behandlungseinrichtungen 8 konstant bleibt. Der Reinraum 6 ist in dieser Figur lediglich schematisch angedeutet. Wie oben beschrieben kann der Reinraum 6 die gesamte Behälterbehandlungsmaschine 1 oder große Teile davon einschließen. Bevorzugt erstreckt sich der Reinraum 6 jedoch (anders als in Fig. 1 angegeben) nur entlang des in dieser Figur nicht gezeigten Transportpfades T und spart große Teile der jeweiligen Transport- und oder Behandlungseinrichtungen 8, wie z.B. deren Antriebe aus. Der Reinraum 6 weist daher abschnittsweise eine ringförmige Gestallt auf.

Wie in Fig. 1 zu erkennen ist, wird der Teilungsabstand 7 vor dem Einbringen der Kunststoffbehältnisse 5 in den Reinraum 6 einmalig eingestellt und über einen langen Abschnitt des Transportpfades nicht verändert. In dem gezeigten Ausführungsbeispiel erfolgen einer oder mehrere Behandlungsschritte außerhalb des Reinraums 6. Nach diesen Behandlungen werden die Behältnisse in einem bestimmten Teilungsabstand 7 für die Übergabe in den Reinraum 6 bereitgestellt. In dem gezeigten Beispiel greift ein Transportstern 4 einzelne Behältnisse und übergibt diese an eine Sterilisationseinrichtung 8a. Der Transportstern 4 weist einen vorgegebenen Abstand zwischen benachbarten auf diesem Transportstern 4 angeordneten Transportelementen 9 auf, durch welche bei dieser Übergabe der später beibehaltene Teilungsabstand 7 vorgegeben wird. Eine diesem ersten Transportstern 4 nachgeschaltete Behandlungseinrichtung 8 ist die Sterilisationseinrichtung 8a. Wie der Figur zu entnehmen ist, weist sie den identischen Teilungsabstand 7 wie der Transportstern 4 auf. Die Dauer eines Umlaufs dieser Sterilisationseinrichtung 8a ist aufgrund der größeren Anzahl an Transportelementen 9 geringer als die eines Umlaufs des Transportsterns 4. So kann die identische Tangentialgeschwindigkeit beider Behandlungseinrichtungen 8 am Punkt der Behältnisübergabe gewährleistet werden. Um eine möglichst lange Verweilzeit und somit Reaktionszeit für den Sterilisationsprozess zur Verfügung zu haben rotiert die Sterilisationseinrichtung 8a in dieser Aufsicht entgegen dem Uhrzeigersinn, wohingegen der Transportstern 4 bevorzugt im Uhrzeigersinn rotiert.

Nach dem Sterilistationsprozess werden die desinfizierten Behältnisse an eine nächste Behandlungseinrichtung 8, über einen zweiten Transportstern 4 übergeben. Auch dieser weist den identischen Teilungsabstand 7 wie die vorherigen Behandlungseinrichtungen 8 auf. Aufgrund der geringeren Anzahl der auf dem Transportstern 4 befindlichen Transportelemente 9 rotiert dieser mit der selben Tangentialgeschwindigkeit wie die vorherigen Behandlungseinrichtungen 8, jedoch mit einer größeren Winkelgeschwindigkeit. Die Rotation erfolgt im Uhrzeigersinn und entspricht damit in der Richtung der des ersten Transportsterns 4.

Stromabwärts dieses zweiten Transportsterns 4 befindet sich eine weitere Behandlungseinrichtung 8, in diesem Fall eine Streckblaseinrichtung 8b. Auch diese weist den selben Teilungsabstand 7 wie die vorherigen Behandlungseinrichtungen 8 auf. Damit können die Behältnisse am Punkt der Behältnisübergabe erneut mit derselben Tangentialgeschwindigkeit bewegt werden, so dass eine besonders reibungslose Übergabe erfolgen kann. Auch diese Behandlungseinrichtung 8 rotiert entgegen dem Uhrzeigersinn, um erneut eine möglichst lange Behandlungszeit während eines Umlaufes zu ermöglichen. Nach der Behandlung, in diesem Fall des Aufblasen des Vorformlings zu einer Flasche, werden die Behältnisse an weitere Behandlungseinrichtungen 8 übergeben, die (und deren Teilungsabstand 7) in der Figur nicht näher dargestellt sind. Prinzipiell ist es möglich eine sehr große Anzahl an Behandlungseinrichtungen 8 so auszuführen, dass sie alle den selben Teilungsabstand 7 aufweisen.

Somit kann ermöglicht werden, dass innerhalb eines großen Bereiches 3, welcher z.B. den gesamten Reinraum 6 umfassen kann, keine weitere Einstellung des Teilungsabstandes 7 notwendig ist. Nach dem Verschließen der Behältnisse unter sterilen Bedingungen können diese aus dem Reinraum 6 ausgeschleust werden und mittels (nicht gezeigten) weiteren Behandlungseinrichtungen 8 (z.B. für die Etikettierung, Qualitätskontrolle, Konfektionierung usw.) behandelt werden.

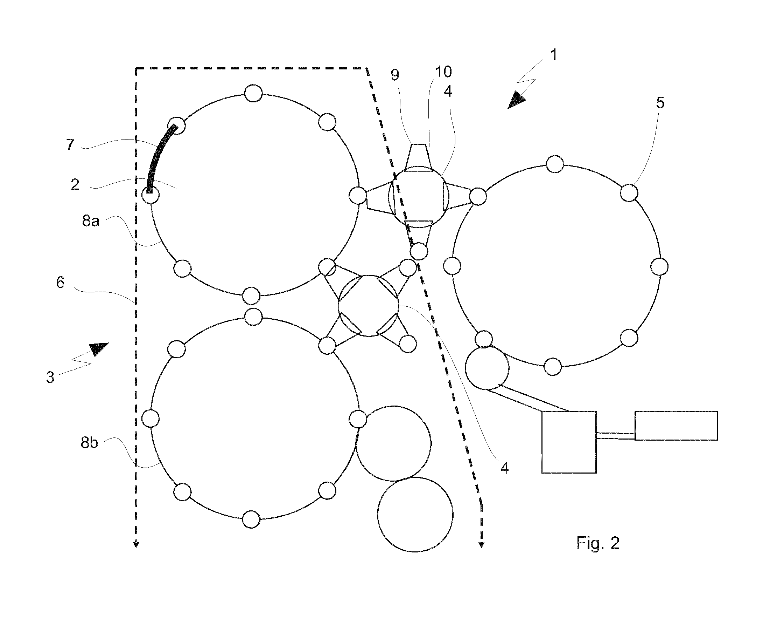

In Figur 2 ist eine weitere schematische Darstellung einer Behälterbehandlungsmaschine 1 ähnlich wie in Fig. 1 gezeigt, bei der jedoch der Teilungsabstand 7 bereits vor der Einbringung Kunststoffbehältnisse 5 in den Reinraum 6 eingestellt wird. Wie auch das in Fig. 1 gezeigte Ausführungsbeispiel weist das in Fig. 2 gezeigte einen Reinraum 6 auf. Der Teilungsabstand 7 zwischen zwei benachbarten Gebinden (Behältnissen) wird jedoch schon vor dem Einschleusen in den Reinraum 6 eingestellt. Die Übergabe zwischen den außerhalb des Reinraums 6 und den innerhalb des Reinraums 6 befindlichen Behandlungseinrichtungen 8 kann somit ebenfalls vereinfacht werden.

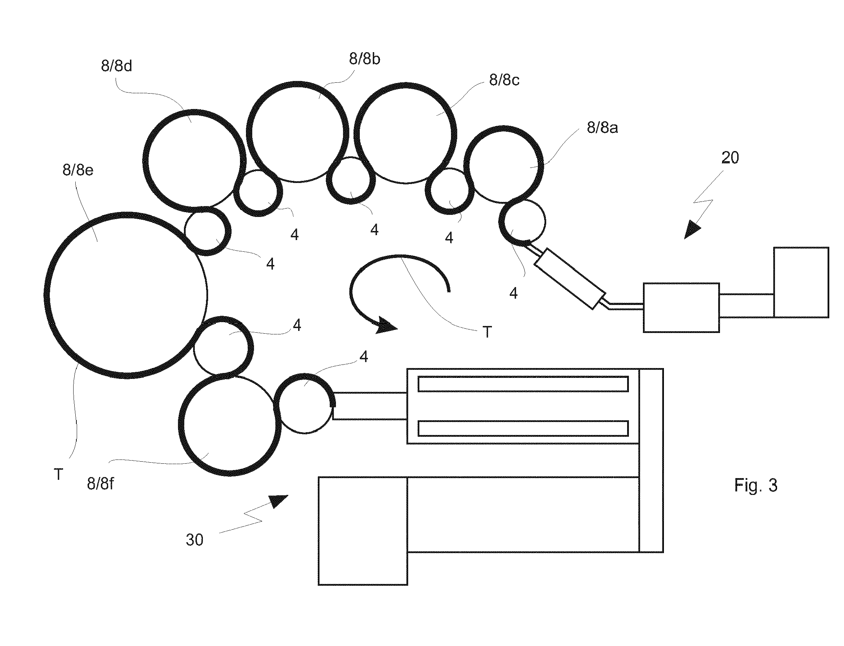

Fig. 3 zeigt eine schematische Darstellung einer Behälterbehandlungsmaschine 1 zum Behandeln von Kunststoffbehältnissen 5, welche eine Vielzahl von Transporteinrichtungen 4 zum Transportieren von Kunststoffbehältnissen 5 umfasst. Dabei kennzeichnen die Bezugszeichen 8a - 8f Behandlungseinrichtungen 8, in denen die Kunststoffbehältnisse 5 oder die entsprechenden Vorformlinge bestimmten Behandlungen unterzogen werden. Ein Reinraum 6 ist in dieser Figur nicht explizit dargestellt, jedoch ist es möglich Teile der Behälterbehandlungsmaschine 1 innerhalb eines Reinraums 6 anzuordnen. Besonders hervorgehoben ist der Transportpfad, entlang dessen die Behältnisse innerhalb der Behälterbehandlungsmaschine 1 transportiert werden. Dieser Transportpfad erstreckt sich annähernd mäanderförmig über eine Vielzahl von Transporteinrichtungen 4 und Behandlungseinrichtungen 8.

Es ist möglich, den Reinraum 6 als Kanal auszuführen, welcher sich entlang des Transportpfades erstreckt. Die jeweiligen Behandlungseinrichtungen 8 sind jeweils mittels Transporteinrichtungen 4 verbunden. Dabei können Transporteinrichtungen 4, welche sich stromaufwärts bezüglich der Sterilisationseinrichtung 8a und stromabwärts der Verschließeinrichtung 8f befinden, je nach Ausführungsform dieser Einrichtungen und Positionierung der Schleuse zwischen sterilem und unsterilem Bereich, auch unsteril ausgeführt sein. Bevorzugt wird nach einer einmaligen Einstellung des Teilungsabstandes 7 im Bereich 3 der Zuführeinrichtung 20 dieser nicht mehr verändert und über den gesamten weiteren Transportpfad beibehalten. Entlang des hervorgehobenen Transportpfades erfolgt keine erneute Einstellung eines anderen Teilungsabstandes 7.

Die Kunststoffbehältnisse 5 oder die entsprechenden Vorformlinge werden in der Sterilisationseinrichtung 8a sterilisiert und ab diesem Verfahrensschritt steril weiter entlang des Transportpfades befördert. In der stromabwärts der Sterilisationseinrichtung 8a folgenden Heizeinrichtung 8c werden die Vorformlinge erwärmt, um anschließend in der weiter stromabwärts folgenden Blaseinrichtung 8b auf das gewünschte Behältnisformat gebracht zu werden. Werden der Sterilisationseinrichtung 8a bereits fertig geformte Behältnisse zugeführt, sind diese Einrichtungen nicht zwangsläufig notwendig. Ebenso optional ist die weiter stromabwärts folgende zweite Sterilisationseinrichtung 8d, welche vor dem folgenden Prozess des Abfüllens des Füllgutes die Behältnisse 5 erneut sterilisiert und diese evtl. auf ein höheres Sterilisationsniveau bringt.

Die Formträger der Blaseinrichtung 8b, welche die Kunststoffvorformlinge aufnehmen, können dabei buchartig um eine vorgegebene Schwenkachse aufklappbar sein. Dabei kann auch eine Formhälfte feststehend (an einem Träger 10) angeordnet sein und die andere dieser gegenüber schwenken. Daneben wäre es auch möglich, dass eine Formhälfte über eine horizontale Schwenkachse (insbesondere nach vorne) aufgeklappt wird oder über eine senkrechte Schwenkachse seitlich weggeschwenkt wird.

Weiter stromabwärts befindet sich die Fülleinrichtung 8e, welcher die Kunststoffbehältnisse 5 erneut mittels einer Transporteinrichtung 4 zugeführt werden. Nach dem Abfüllvorgang werden die noch unverschlossenen Behältnisse 5 ebenfalls mittels einer Transporteinrichtung 4 von der Fülleinrichtung 8e an eine Verschließeinrichtung 8f übergeben. In dieser Verschließeinrichtung 8f erfolgt ebenfalls unter sterilen Bedingungen das Verschließen der Behältnisse 5, wodurch der abgefüllte Inhalt ebenfalls steril gehalten wird. Wie bereits oben erwähnt kann der anschließende Abtransport der befüllten und verschlossenen Kunststoffbehältnisse 5 von der Verschließeinrichtung 8f theoretisch mittels einer unsterilen Transporteinrichtung 4 erfolgen.

Je nach Ausführungsform der Verschließeinrichtung 8f und dem Übergang zwischen sterilem und unsterilem Bereich kann jedoch auch eine sterile Transporteinrichtung 4 zum Abtransport der befüllten und verschlossenen Kunststoffbehältnisse 5 notwendig sein. Beispielsweise kann der Transport unter sterilen Bedingungen zu einer Schleuse erfolgen, welche den Übergang zwischen sterilem und unsterilem Bereich darstellt. Anschließend erfolgt die Übergabe an eine nicht näher beschriebene Abführeinrichtung 30 in welcher nachfolgende Behandlungsschritte wie beispielsweise die Etikettierung, Palettierung o.ä. unter unsterilen Bedingungen erfolgen können.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Behälterbehandlungsmaschine
- 2: Streckblaseinrichtung
- 3: Bereich
- 4: Transporteinrichtung / Transportstern
- 5: Kunststoffbehältnis
- 6: Reinraum
- 7: Teilungsabstand
- 8: Behandlungseinrichtung
- 8a: Sterilisationseinrichtung
- 8b: Streckblaseinrichtung
- 8c: Heizeinrichtung / Ofen
- 8d: zweite Sterilisationseinrichtung
- 8e: Fülleinrichtung
- 8f: Verschließeinrichtung
- 9: Transportelement
- 10: Träger des Transportelements
- 20: Zuführeinrichtung
- 30: Abführeinrichtung
- R: radiale Richtung
- T: Transportrichtung

## Patentansprüche

1. Behälterbehandlungsmaschine (1) mit einer Streckblaseinrichtung (2) zum Umformen von Kunststoffbehältnissen (5), welche mindestens drei Transporteinrichtungen (4) zum Transportieren von Kunststoffbehältnissen (5) aufweist, wobei jede Transporteinrichtung (4) eine Vielzahl von Transportelementen (9) aufweist, welche jeweils an einem Träger (10) angeordnet sind und welche dazu geeignet sind, die Kunststoffbehältnisse (5) zu transportieren,
**dadurch gekennzeichnet, dass**
die Behälterbehandlungsmaschine (1) einen Bereich (3) aufweist, welcher sich über mindestens drei Transporteinrichtungen (4) erstreckt, in welchem die Transportelemente (9) jeder der in diesem Bereich (3) angeordneten Transporteinrichtungen (4) in äquidistanten Abständen zu jeweils benachbarten Transportelementen (9) der selben Transporteinrichtung (4) angeordnet sind.

2. Behälterbehandlungsmaschine (1) nach Anspruch 1,
**dadurch gekennzeichnet, dass**
der Bereich (3) ein Reinraum (6) ist.

3. Behälterbehandlungsmaschine (1) nach Anspruch 1 oder 2,
**dadurch gekennzeichnet, dass**
eine Transporteinrichtung (4) und eine stromabwärts unmittelbar folgende Streckblaseinrichtung (2) Transportelemente (9) in äquidistanten Abständen zu jeweils benachbarten Transportelementen (9) aufweisen.

4. Behälterbehandlungsmaschine (1) nach wenigstens einem der vorangegangenen Ansprüche,
**dadurch gekennzeichnet, dass**
die Behälterbehandlungsmaschine (1) mindestens eine Heizeinrichtung zum Erwärmen der Kunststoffbehältnisse (5) umfasst.

5. Behälterbehandlungsmaschine (1) nach wenigstens einem der vorangegangenen Ansprüche,
**dadurch gekennzeichnet, dass**
die Behälterbehandlungsmaschine (1) mindestens eine Sterilisationseinrichtung zum Sterilisieren der Kunststoffbehältnisse (5) umfasst.

6. Behälterbehandlungsmaschine (1) nach wenigstens einem der vorangegangenen Ansprüche,
**dadurch gekennzeichnet, dass**
mindestens eine der Transporteinrichtungen (4) dazu geeignet ist, die Kunststoffbehältnisse (5) entlang eines Abschnittes eines weitgehend kreisrunden Transportpfades zu transportieren.

7. Behälterbehandlungsmaschine (1) nach wenigstens einem der vorangegangenen Ansprüche,
**dadurch gekennzeichnet, dass**
ein Transportpfad, entlang welchem die Kunststoffbehältnisse (5) transportierbar sind, einen Abschnitt des Umfanges mindestens einer Transporteinrichtung (4) umfasst, welcher durch einen Winkel von über 180° aufgespannt ist.

8. Behälterbehandlungsmaschine (1) nach wenigstens einem der vorangegangenen Ansprüche,
**dadurch gekennzeichnet, dass**
mindestens eines der Transportelemente (9) in seinem Öffnungsquerschnitt zum Einführen und/oder Greifen eines Kunststoffbehältnisses (5) veränderbar ist.

9. Behälterbehandlungsmaschine (1) nach wenigstens einem der vorangegangenen Ansprüche
**dadurch gekennzeichnet, dass**
die Abstände der Transportelemente (9) einer ersten in dem Bereich (3) angeordneten Transporteinrichtung (4) in einem ganzzahligen Verhältnis zu den Abständen der Transportelemente (9) einer zweiten in dem Bereich (3) angeordneten Transporteinrichtung (4) stehen.

10. Behälterbehandlungsmaschine (1) nach Anspruch 9,
**dadurch gekennzeichnet, dass**
wenigstens zwei Transporteinrichtungen (4) in dem Bereich (3) unterschiedliche und in einem ganzzahligen Verhältnis zueinander stehende Transportgeschwindigkeiten aufweisen.

11. Verfahren zum Transportieren von Kunststoffbehältnissen (5) in einer Behälterbehandlungsmaschine (1), insbesondere Streckblasmaschine, mittels einer Transporteinrichtung (4) mit einer Vielzahl von Transportelementen (9), welche an einem Träger (10) angeordnet sind und welche die Kunststoffbehältnisse (5) transportieren, **dadurch gekennzeichnet, dass**
die Kunststoffbehältnisse (5) in einen sich über mindestens drei Transporteinrichtungen (4) erstreckenden Bereich (3) der Behälterbehandlungsmaschine (1) mittels einer Vielzahl von in äquidistanten Abständen zu jeweils benachbarten Transportelementen (9) der selben Transporteinrichtung (4) angeordneten Transportelementen (9) so transportiert werden, dass der Abstand zwischen zwei benachbarten Kunststoffbehältnissen (5) konstant bleibt.

12. Verfahren nach Anspruch 11,
**dadurch gekennzeichnet, dass**
die Kunststoffbehältnisse (5) in einem Bereich (3) der Behälterbehandlungsmaschine (1), welcher eine Heizeinrichtung für Kunststoffbehältnisse (5) aufweist, in einem konstanten, bevorzugt äquidistanten Abstand, entlang eines Transportpfades transportiert werden.

13. Verfahren nach Anspruch 11,
**dadurch gekennzeichnet, dass**
die Kunststoffbehältnisse (5) innerhalb des Bereiches (3) unter sterilen Bedingungen innerhalb eines Reinraums (6), transportiert werden.

14. Verfahren nach Anspruch 11 oder 12,
**dadurch gekennzeichnet, dass**
ein Abstand zwischen den Kunststoffbehältnissen (5) stromabwärts des Bereiches (3) in dem der Abstand zwischen zwei benachbarten Kunststoffbehältnissen (5) während des Transportes über mehrere Transporteinrichtungen (4) konstant bleibt, mittels einer Abstandserzeugungseinrichtung (5), bevorzugt mittels einer Schnecke, verändert wird.
